# FASCICULE DE BREVET EUROPEEN

(11) **EP 1 495 119 B1**
(45) Date de publication et mention de la délivrance du brevet: **17.01.2007**
(21) Numéro de dépôt: 03745840.3
(22) Date de dépôt: 09.04.2003
(51) Int. Cl.: C12N 15/10, C12M 1/12, C12Q 1/68, C07H 1/08

(54) **PROCEDE ET AUTOMATE D EXTRACTION D ACIDES NUCLEIQUES A PARTIR D UN MELANGE COMPLEXE**
VERFAHREN UND AUTOMATISCHE EXTRAKTION VON NUCLEINSÄUREN AUS KOMPLEXEN MISCHUNGEN
METHOD AND APPARATUS FOR EXTRACTING NUCLEIC ACIDS FROM A COMPLEX MIXTURE

(30) Priorité: 09.04.2002 FR 0204424
(43) Date de publication de la demande: 12.01.2005
(73) Titulaire: Genesystems, 35170 Bruz (FR)
(72) Inventeur: HALLIER-SOULIER, Sylvie, 35170 Bruz (FR); LEBORGNE, Ga¬lle, 35700 Rennes (FR); BATISSE-DEBITTE, Nadine, 35230 Saint-Erblon (FR); FESTOC, Gabriel, 35000 Rennes (FR); BADICHE, Xavier, 75014 Paris (FR)
(74) Mandataire: Desaix, Anne
(86) Numéro de dépôt international: PCT/FR2003/001123
(87) Numéro de publication internationale: WO 2003/085109

(56) Documents cités:
- EP-A- 0 245 945
- WO-A-00/21973
- WO-A-02/16383
- WO-A-93/01312
- WO-A-94/21780
- WO-A-95/14086
- WO-A-99/14309
- US-A- 5 187 083
- KUSKE CHERYL R ET AL: "Small-scale DNA sample preparation method for field PCR detection of microbial cells and spores in soil." APPLIED AND ENVIRONMENTAL MICROBIOLOGY, vol. 64, no. 7, juillet 1998 (1998-07), pages 2463-2472, XP002226433 ISSN: 0099-2240 cité dans la demande

## Description

La présente invention relève du domaine de l'analyse d'échantillons biologiques et, plus particulièrement, de l'analyse du contenu bactérien de mélanges complexes, notamment d'échantillons alimentaires.

L'invention concerne un procédé d'extraction des acides nucléiques de microorganismes contenus dans un mélange complexe. Un tel procédé comprend au moins les étapes suivantes :
(12) la rétention des microorganismes contenus dans le mélange sur un dispositif de type cartouche de filtration (40) ;
(14) la lyse des microorganismes *in situ* ; et
(16) la récupération des acides nucléiques à partir de la cartouche de filtration (40) sur un adaptateur contenant un filtre de clarification, lesdits acides nucléiques étant récupérés sous la forme d'un lysat filtré.

La présente invention a en outre pour objets une cartouche de filtration ainsi qu'un automate d'extraction des acides nucléiques de microorganismes contenus dans un mélange complexe ou dans de l'eau, lesdits cartouche et automate étant utiles pour la mise en oeuvre du procédé d'extraction selon l'invention.

L'invention concerne enfin un dispositif pour l'extraction et la détection et/ou la quantification des acides nucléiques de microorganismes contenus dans un mélange complexe, ledit dispositif comprenant un automate d'extraction d'acides nucléiques conforme à l'invention, couplé à un automate d'amplification en chaîne thermo-dépendante desdits acides nucléiques.

Le comportement actuel du public, représentant les consommateurs potentiels, est symptomatique d'une préoccupation de santé et sécurité générales, en ce que sont exigées toujours plus de garanties sur l'innocuité, les qualités et la provenance des produits disponibles sur le marché. Du reste, ce phénomène a pris beaucoup d'ampleur ces dernières années et continue d'aller croissant. Aussi convient-il d'apporter des réponses solides au public afin de satisfaire son besoin de certitudes et d'assurances.

A cet égard, les pouvoirs publics et autres autorités et organismes compétents en matière de santé publique et vigilance sanitaire imposent des contrôles réguliers afin de veiller au strict respect des normes de sécurité et de qualité, notamment dans le domaine de l'alimentation par les producteurs, fabricants, distributeurs et restaurateurs. La qualité du contrôle microbiologique est un point crucial auquel il convient de veiller tout au long de la chaîne entre le producteur et le consommateur.

Le principe de tels contrôles repose sur des méthodes d'analyse fondées sur des techniques courantes de chimie ou biochimie analytique, parmi lesquelles la chromatographie, la résonance magnétique nucléaire et la spectrométrie de masse, ainsi que des techniques de biologie moléculaire permettant, par exemple, de détecter des acides nucléiques spécifiques d'organismes notamment pathogènes par PCR ou hybridation à l'aide de sondes.

Ces contrôles peuvent être effectués à grande échelle, notamment au niveau d'une exploitation agricole ou d'une industrie de production. De plus, ils sont parfois pratiqués dans l'urgence. En l'occurrence lorsque les produits suspects sont déjà disponibles à la vente, il convient d'évaluer l'innocuité desdits produits dans les plus brefs délais afin, le cas échéant, d'ordonner leur retrait du marché avant une éventuelle contamination de masse.

En particulier, les contrôles sanitaires sont pratiqués dans le cadre de l'évaluation du niveau de contamination d'aliments à risque, tels les viandes, poissons, laitages et aliments susceptibles d'avoir subi des altérations résultant d'éventuelles variations sensibles de température lors du franchissement des différentes étapes de la « chaîne du froid », ainsi qu'en matière de préservation et surveillance de l'environnement, s'agissant par exemple du contrôle du niveau de pollution des sources et réseaux urbains d'eau potable.

Selon des applications plus médicales, la pureté des extraits d'acides nucléiques issus de prélèvements biologiques de patients ou sujets à risque est un critère essentiel pour la mise en oeuvre de méthodes diagnostiques et notamment, la réalisation de tests de dépistage ou prédisposition vis-à-vis de maladies génétiques.

En tout état de cause, les prélèvements effectués *in situ* doivent être traités dans les plus brefs délais tout en assurant efficacité et sécurité optimales.

L'une des premières étapes du traitement des prélèvements biologiques peut notamment consister à manipuler *in vitro* les acides nucléiques à des fins d'analyse qualitative et/ou quantitative et plus précisément, à isoler lesdits acides nucléiques de leur environnement cellulaire.

Les acides nucléiques, ADN et ARN, sont la cible d'interactions multiples, liées aux processus biologiques dans lesquels lesdits acides nucléiques jouent un rôle fondamental. A titre d'exemple, ces derniers interagissent directement avec les enzymes responsables de l'expression des gènes, ainsi qu'avec les facteurs protéiques de régulation de ladite expression, celle-ci constituant un processus biologique fondamental contribuant à la survie, au développement et au renouvellement des cellules.

De tels complexes associant les acides nucléiques d'intérêt à des protéines et/ou d'autres acides nucléiques compliquent considérablement l'isolement et la purification des seuls acides nucléiques recherchés.

Afin de surmonter cette difficulté, les méthodes conventionnelles d'extraction des acides nucléiques sacrifient généralement le temps de mise en oeuvre au profit du degré de pureté des extraits obtenus. En principe, de telles méthodes comprennent au moins les trois étapes suivantes : (i) la lyse des enveloppes cellulaires permettant de libérer les acides nucléiques dans le milieu ; (ii) la dénaturation des complexes acides nucléiques-protéines ; et (iii) la séparation des acides nucléiques d'intérêt des autres macromolécules (Sambrook et Russel. 2001. Molecular cloning : a laboratory manual, 3^{rd} ed. Cold Spring Harbor Laboratory Press, Cold Spring Harbor, N.Y.)

Ces méthodes conventionnelles, bien que conservant pour la plupart la trame représentée par les trois étapes énoncées *supra,* ont été empiriquement modifiées en fonction des différents objectifs à atteindre. Ainsi, leur mise en oeuvre diffère sensiblement selon que le but poursuivi consiste à isoler (i) les ADN et/ou les ARN, (ii) à partir de cellules d'organismes procaryotes ou eucaryotes, sachant que dans le cas des organismes procaryotes, il convient le cas échéant de (iii) discriminer les ADN de nature plasmidique ou chromosomique.

Dans la trame sus-visée, figure une étape essentielle consistant à extraire les acides nucléiques au moyen de solvants organiques, tels le phénol, le chloroforme, l'alcool isoamylique, ou un mélange de ceux-ci. Les acides nucléiques recherchés, contenus dans la phase aqueuse, sont alors recueillis après séparation des phases organique et aqueuse par centrifugation.

La mise en oeuvre d'une telle étape représente une, si ce n'est la limitation principale des procédures d'extraction des acides nucléiques selon l'art antérieur. En effet, la centrifugation nécessaire à la séparation des phases organique et aqueuse constitue un obstacle majeur à l'automatisation de la méthode en imposant d'une part, l'intervention nécessaire de l'expérimentateur et d'autre part, des coûts élevés de mise en oeuvre du fait d'installations coûteuses et volumineuses.

En définitive, la plupart des méthodes conventionnelles de lyse cellulaire permettent d'extraire les acides nucléiques, non pas sous leur forme native, mais sous la forme de fragments de grande taille (50 kb). La purification subséquente des extraits d'acides nucléiques à l'aide de solvants organiques, tel un mélange phénol-chloroforme, peut entraîner un cisaillement desdits acides nucléiques, générant alors des fragments de taille hétérogène et inférieure à 50 kb.

Un tel « artéfact expérimental » est susceptible d'empêcher ou fausser l'interprétation des résultats, en particulier lorsque l'homme du métier discrimine les produits issus de l'extraction sur la seule base de leur taille, au moyen de techniques d'électrophorèse sur gels par exemple, ce dernier se trouvant alors confronté à des phénomènes dits de « smear » qui font apparaître les fragments d'acides nucléiques non pas comme des bandes parfaitement individualisées, mais comme des tâches dans lesquelles sont regroupées plusieurs bandes correspondant à plusieurs fragments d'acides nucléiques. Cet « artéfact » peut également entraîner une réduction sévère de sensibilité lors de la détection des acides nucléiques ainsi extraits par des méthodes d'hybridation, notamment sur membranes.

Pour remédier à cet inconvénient de l'état de la technique, un automate d'extraction et purification d'acides nucléiques à partir de cellules a été décrit dans le brevet EP 0 245 945. Cet automate permet de réaliser l'étape d'extraction au moyen d'un solvant organique à base de phénol et ce, en s'affranchissant des multiples centrifugations jusque-là nécessaires. Brièvement, une émulsion est générée par l'addition de solvant au lysat cellulaire, ladite émulsion étant chauffée pour entraîner la séparation des phases. Une étape d'extraction finale au chloroforme est réalisée.

Nonobstant sa possible automatisation, cette méthode ne résout pas le problème posé par l'art antérieur, et notamment lié à l'utilisation de solvants organiques. Bien que l'extraction au moyen desdits solvants élimine efficacement les contaminants, à savoir les détergents utilisés dans l'étape de lyse des cellules ainsi que les protéines, ils restent néanmoins fortement toxiques. En conséquence, leur manipulation nécessite vigilance et parcimonie, et doit être confinée au niveau de postes de sécurité dotés de systèmes de ventilation appropriés.

Afin d'obvier aux inconvénients évoqués *supra* et présentés par les méthodes d'extraction des acides nucléiques de l'état de la technique, lesdites méthodes étant difficilement conciliables avec les impératifs de sécurité, efficacité et rentabilité de l'industrie ou du laboratoire d'analyses moderne, une approche alternative a été décrite dans la demande internationale WO 93/01312.

Cette demande divulgue une cartouche de préparation d'acides nucléiques, notamment d'ADN génomique, à partir d'un échantillon de sang, de cellules de tissus ou de cellules en culture, autrement dit des suspensions de cellules eucaryotes relativement homogènes, dans un fluide biologique ou un milieu de culture.

La demande internationale WO 93/01312 décrit en outre un dispositif et un procédé utilisant ladite cartouche.

Ainsi, la cartouche comprend notamment une enceinte de dialyse délimitée par des membranes de dialyse. Le cas échéant, la cartouche comprend en outre un filtre de rétention des noyaux des cellules.

La demande internationale WO 93/01312 propose un procédé de préparation, c'est-à-dire d'isolement et purification des acides nucléiques à partir d'un échantillon contenant des cellules eucaryotes, par lyse des noyaux, dégradation des protéines et purification desdits acides nucléiques par dialyse.

La technologie relative à la cartouche d'extraction décrite dans la demande internationale WO 93/01312 n'est cependant pas adaptée à une extraction des acides nucléiques de procaryotes présents, le cas échéant, dans des mélanges complexes hétérogènes, tels que des échantillons alimentaires bruts, non préalablement modifiés par exemple par dessiccation, et dont les constituants sont pour partie solides et pour partie liquides.

D'autres méthodes d'extraction d'acides nucléiques à partir de leucocytes, mais également applicables à d'autres types cellulaires telles que les bactéries, sont rapportées dans les demandes de brevet WO 00/21973 et WO 02/16383. Ainsi, ces deux demandes divulguent une méthode d'extraction qui comprend les étapes suivantes :
(a) le dépôt d'un échantillon sur un filtre permettant de retenir les cellules contenues dans cet échantillon (rétentat), et l'élimination des contaminations,
(b) la lyse du rétentat *in situ* et formation d'un lysat cellulaire,
(c) la filtration du lysat cellulaire et rétention des acides nucléiques sur le filtre et élimination du reste du lysat cellulaire,
(d) éventuellement, le lavage des acides nucléiques contenus dans le filtre, et
(e) l'élution des acides nucléiques,
dans laquelle la composition et les dimensions du filtre sont choisis de manière à ce que le filtre soit capable de retenir les cellules et les acides nucléiques.

De plus, la demande WO 94/21780 décrit une méthode pour dénombrer les microorganismes présents dans un échantillon par fixation de ces microorganismes sur un filtre et leur lyse in situ. Dans un mode de réalisation particulier, après lyse des microorganismes, ceux-ci sont détectés par amplification par PCR directement sur le filtre.

Enfin, une autre demande de brevet, WO 99/14309 propose la détection de différents microorganismes après leur filtration sur un filtre de nitrocellulose présentant des pores de 0,45 µm.

En l'espèce, l'invention vise à proposer un procédé d'extraction d'acides nucléiques de procaryotes automatisable et adapté au traitement d'un milieu de départ complexe.

Ainsi, le procédé selon l'invention, entièrement automatisable, répond ainsi à un souci de rationalisation et d'économie en ce qu'il : (i) est directement applicable à l'extraction d'acides nucléiques à partir de prélèvements bruts, quand bien même ceux-ci seraient complexes et hétérogènes en termes de composition et structure ; (ii) permet une extraction rapide, réalisable en moins de 3 heures ; (iii) fournit des résultats reproductibles ; (iv) permet de traiter simultanément jusqu'à six échantillons différents, tout en assurant leur traçabilité à chaque étape ; (v) peut être mis en oeuvre par du personnel non qualifié ; (vi) ne requiert ni intervention ni surveillance de la part de l'expérimentateur, si ce n'est éventuellement de manière très ponctuelle, ledit expérimentateur se trouve alors disponible pour effectuer, en parallèle des extractions en cours, d'autres opérations ou travaux ; et (vii) peut être mis en oeuvre en routine à l'échelle du laboratoire d'analyses ou de l'industrie, sans nécessiter de lourds investissements en matière d'équipements.

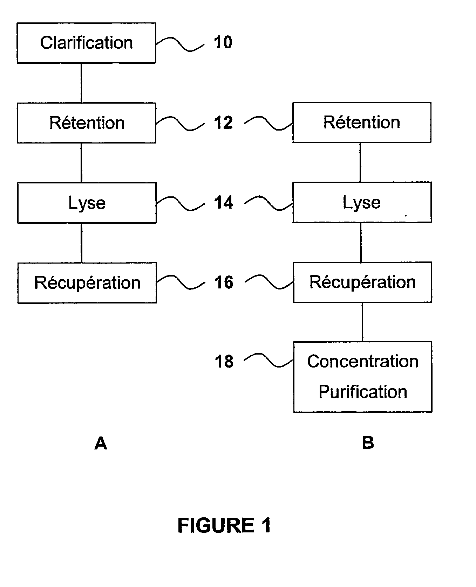

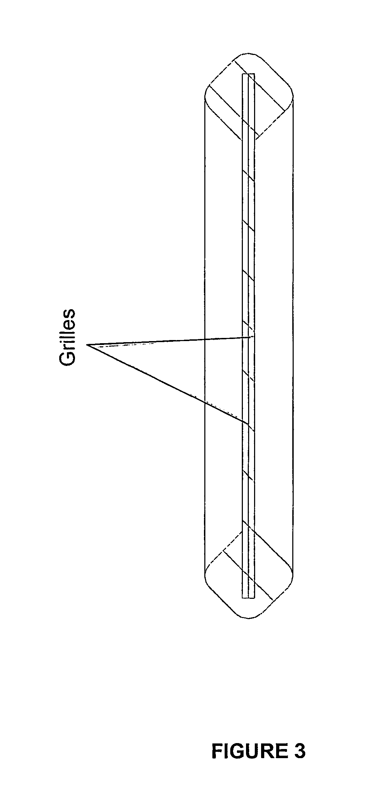

L'invention concerne donc un procédé d'extraction des acides nucléiques de microorganismes contenus dans un mélange complexe, notamment alimentaire, ledit procédé comprenant au moins les étapes suivantes :
(12) la rétention des microorganismes contenus dans le mélange sur un dispositif de type cartouche de filtration 40 (cf. Figure 3)
(14) la lyse des microorganismes *in situ*
(16) la récupération des acides nucléiques à partir de la cartouche de filtration 40 (Figure 1).
Selon la nature de l'échantillon à analyser (broyat alimentaire solide/liquide
ou eau, par exemple eau de consommation), une première étape est requise :
(10) la clarification dudit mélange par filtration sur un dispositif de type cartouche de préfiltration (Figure 1, voie A), l'étape (12) étant alors réalisée sur le filtrat
Dans le cas où les acides nucléiques sont extraits en vue d'une quantification, une étape supplémentaire est requise :
(18) la concentration et la purification des acides nucléiques par chromatographie d'adsorption liquide-solide (Figure 1, voie B).

Il est fait référence au niveau des étapes 12, 14 et 16 à un « dispositif de type cartouche de filtration » 40 (Figure 3). L'on entend par une telle désignation, ainsi que par les termes et expressions équivalents « cartouche », « cartouche d'extraction » et « cartouche de filtration », un système comprenant un filtre de rétention 42 serré entre deux grilles ou collé à une seule grille, la (les) grille(s) étant maintenue(s) par tout moyen, par exemple par deux bagues collées, vissées ou clipsées, ou étant enchâssée(s) dans une bague.

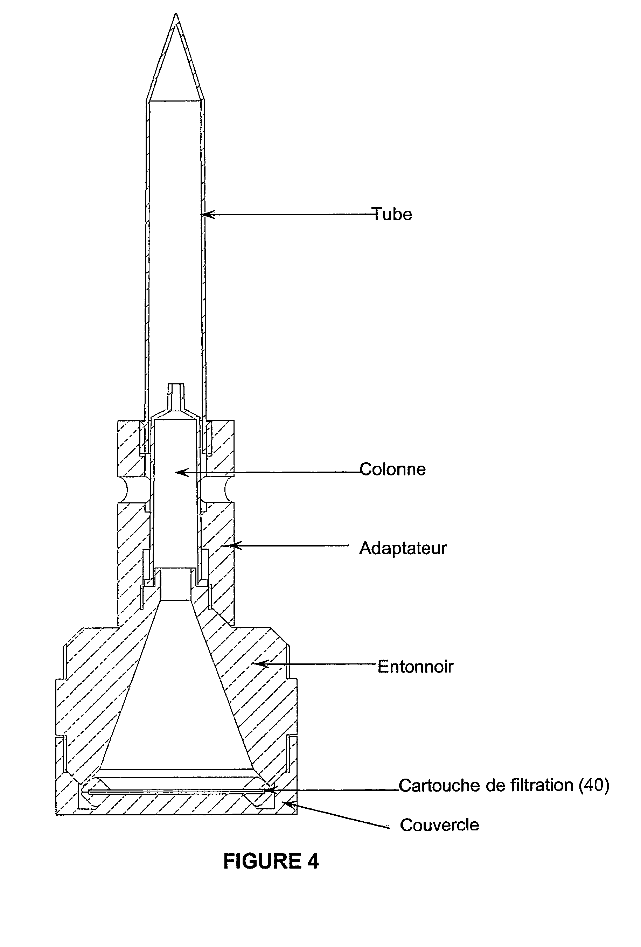

Cette cartouche peut être soumise aux traitements de lyse (Figure 4). Dans ce cas, la pièce en plastique sur laquelle est posée la cartouche de filtration est dévissée du poste de filtration et la cartouche est fermée de façon étanche sur sa face inférieure - c'est-à-dire la face qui n'est pas en contact avec les bactéries - par un couvercle. La face supérieure de la cartouche de filtration est fermée de façon étanche par un dispositif comprenant un entonnoir, un adaptateur, un filtre de clarification du lysat et un tube de 15 ml à fond conique. L'adaptateur est vissé sur l'entonnoir. Il contient le filtre de clarification du lysat, préalablement emboîté dans l'adaptateur. A l'extrémité libre de l'adaptateur, un tube collecteur de 15 ml à fond conique est vissé, tel que représenté sur la Figure 4.

Ledit système présente des spécificités originales du fait des caractéristiques des filtres le composant. En effet, comme le montre la description détaillée de l'invention ci-après, la sélection des filtres est un élément essentiel d'obtention du résultat escompté. Une description de la cartouche de filtration 40 objet de la présente invention est fournie infra.

Au sens de la présente invention, l' « extraction » des acides nucléiques comprend à la fois l'isolement desdits acides nucléiques de leur environnement biologique naturel, leur purification et leur concentration. Le degré de pureté et la concentration des acides nucléiques extraits à l'aide du procédé selon l'invention sont tels que lesdits acides nucléiques peuvent faire l'objet d'une détection par des méthodes aussi sensibles que la PCR et ce, directement et sans n'avoir à subir aucun traitement supplémentaire d'élimination des contaminants résiduels susceptibles d'inhiber ou altérer la spécificité et la sensibilité desdites méthodes, et notamment des réactions de PCR.

Les « acides nucléiques » comprennent, conformément à l'acception usuelle, les ADN et les ARN. Il est évident que ledit procédé peut être utilisé aussi bien dans le cadre de l'extraction des seuls ADN, moyennant l'addition de RNAses à une étape que l'homme du métier saura choisir sans difficultés, que des seuls ARN, grâce à l'addition de DNAses et d'antiRNAses, que des ADN et ARN.

Par « acides nucléiques d'intérêt », « recherchés », « désirés », ou tout autre qualificatif équivalent, l'homme du métier identifiera aisément les acides nucléiques cibles du procédé d'extraction selon l'invention, lesdits acides nucléiques constituant en tant que tels le matériel de départ de la procédure d'analyse qualitative et/ou quantitative subséquente.

L'on entend ici par « microorganisme », tout organisme procaryote, cette définition englobant aussi bien les bactéries à Gram positif et négatif que celles dépourvues de paroi, ainsi que les formes sporulées des bactéries.

Par « mélange complexe », il est fait référence au sens de la présente invention à toute composition contenant des cellules d'organismes procaryotes, ladite composition pouvant être de nature hétérogène, une illustration étant un échantillon alimentaire brut caractérisé en ce qu'il comprend un plus ou moins grand nombre d'éléments différents, dont certains sont à l'état liquide et d'autres solide, ou de nature plus homogène, correspondant alors, par exemple, à une culture bactérienne plus ou moins pure.

Le procédé d'extraction selon l'invention fait appel aux techniques de séparation à membranes ou de filtration, lesquelles utilisent des membranes poreuses dites permsélectives en référence à leurs propriétés sélectives et dont le fonctionnement est assimilable à une barrière à effet tamis : les molécules ou particules dont la taille approximative est supérieure à un seuil donné, correspondant à la taille moyenne des pores de la membrane, sont retenues tandis que celles dont la taille est inférieure au dit seuil passent au travers de la « barrière », sous l'action d'un flux de solvant généré par l'application d'un gradient de pression.

A cet égard, le passage à travers le tamis constitué par la membrane poreuse permet de séparer deux liquides, le perméat ou filtrat qui est passé à travers les pores de ladite membrane, et le rétentat qui est enrichi par rapport au liquide initial en espèces de diamètre moyen supérieur à celui des pores.

Parmi les techniques de filtration, il y a lieu de distinguer notamment l'ultrafiltration et la microfiltration.

L'ultrafiltration utilise des membranes microporeuses dont le diamètre des pores est généralement compris entre 1 et 100 nm. De telles membranes laissent passer les petites molécules (eau, sels) et retiennent les molécules de masse molaire élevée (polymères, protéines, colloïdes). Nonobstant l'intervalle relatif au diamètre des pores indiqué *supra,* il est d'usage d'utiliser, pour caractériser la membrane dans le domaine de l'ultrafiltration, la notion de seuil de coupure plutôt que celle de taille des pores. En effet, le seuil de coupure est défini comme la masse, exprimée en daltons, de la molécule retenue à 90 voire 95 % par la membrane en question.

Quant à la microfiltration, elle consiste en un procédé de séparation solide-liquide mettant en oeuvre des membranes dont le diamètre moyen des pores est compris entre 0,1 et 10 µm environ. Ce procédé permet donc de retenir les particules en suspension.

Les procédés de filtration peuvent être mis en oeuvre selon différents modes et configurations, le type d'écoulement et la géométrie du dispositif hébergeant la membrane ayant un effet déterminant sur le rendement de la séparation. Des exemples de modes et configurations possibles sont donnés ci-après à titre indicatif.

En filtration frontale, le liquide contenant les espèces à séparer est amené perpendiculairement à la membrane tandis qu'en mode tangentiel, la membrane poreuse est balayée tangentiellement par ledit liquide.

La configuration plane facilite les opérations de montage/démontage et est utilisable avec des fluides visqueux. Cependant, le risque d'émergence de phénomènes limitants tels que définis infra, dont en particulier le colmatage, doit être considéré lors de la mise en oeuvre du procédé de filtration en configuration plane.

La configuration en spirale consiste en une membrane plane enroulée sur elle-même. Le filtrat est alors collecté au niveau de la lumière centrale. Cette configuration est avantageuse du point de vue de sa compacité, mais rend le nettoyage de la membrane ardu et le démontage du système de filtration impossible. De plus, elle génère des phénomènes de colmatage non négligeables.

Selon la configuration tubulaire, la membrane tapisse l'intérieur d'une matrice cylindrique en acier poreux ou en fibre de verre. L'alimentation en solution à filtrer se fait par l'intérieur de la matrice, ladite solution traversant ensuite la membrane sous l'effet de la pression et le filtrat étant alors récupéré à l'extérieur de la matrice. Ce système présente l'avantage d'un nettoyage aisé par simple inversion du flux de filtration mais exige des volumes d'installations importants.

Le choix des conditions opératoires du procédé de filtration est généralement régi par le souci de réduire les phénomènes limitants, parmi lesquels l'encrassement de la membrane, ou colmatage, et la polarisation de concentration.

Le colmatage consiste en des phénomènes physiques, chimiques et biologiques qui se produisent à l'interface membrane-solution. Il se caractérise par une modification des propriétés filtrantes de la membrane lors de son utilisation du fait notamment de phénomènes de dépôt et/ou d'adsorption provoquant une accumulation de matière dans les pores, puis à la surface de ladite membrane. Le colmatage a pour conséquence immédiate l'obstruction des pores, ce qui entraîne des variations à la fois de perméabilité et de sélectivité dont l'impact sur l'efficacité de la séparation s'avère attentatoire et parfois dramatique.

Les membranes utilisées ayant la propriété d'effectuer des séparations à l'échelle moléculaire ou particulaire, l'accumulation des espèces (molécules ou particules), progressivement ralenties puis arrêtées à la surface desdites membranes, est un phénomène inéluctable, inhérent aux procédés de filtration. Ledit phénomène, appelé polarisation de concentration, génère un flux de diffusion de la membrane vers le coeur de la solution à traiter, c'est-à-dire en direction opposée à celle du flux de filtration que l'expérimentateur souhaite imposer à ladite solution. La polarisation de concentration induit en conséquence une diminution du flux de filtration, une variation de sélectivité, le dépôt lié à l'accumulation jouant éventuellement le rôle de « seconde membrane », et un colmatage des pores de la membrane du fait de précipitations ou gélifications superficielles, les espèces accumulées formant des réseaux d'aspect quasi-solide.

Au cours de l'étape 10 du procédé d'extraction selon l'invention, (qui n'est pas indispensable lorsque le matériel à analyser est de l'eau), le matériel de départ, à savoir le mélange complexe, est filtré afin de retenir les particules dont le diamètre moyen des pores est supérieur à environ 5 µm, et de préférence supérieur à environ 8 µm.

L'on entend par « membrane » ou « filtre » au sens de la présente invention, tout dispositif conforme à la définition de « membrane permsélective » telle qu'énoncée *supra.*

Le procédé de filtration mis en oeuvre dans l'étape 10 est de type microfiltration frontale, ladite microfiltration étant réalisée en mode plan, sous vide.

Les paramètres de mise en oeuvre de cette étape de microfiltration ont été dictés par le double objectif à atteindre, à savoir d'une part discriminer par la taille les débris contenus dans le mélange complexe et comprenant éventuellement des cellules eucaryotes, qu'il convient d'éliminer, et les cellules procaryotes, pour lesquelles le niveau de perte toléré doit être minimal, et d'autre part éviter tout phénomène limitant tel que le colmatage et la polarisation de concentration.

A cette fin, l'étape 10 est réalisée à l'aide d'un filtre constitué d'un matériau adapté au traitement des liquides visqueux fortement chargés en particules, tels le polycarbonate et le nitrate de cellulose, ledit filtre étant à usage unique afin d'éviter tout risque de contamination entre les différents échantillons susceptibles d'être traités simultanément. Le diamètre moyen des pores dudit filtre est compris entre 1,2 et 12 µm, et de préférence sensiblement égal à 5µm.

L'étape 12 du procédé d'extraction objet de la présente invention consiste en une microfiltration réalisée en mode plan, tangentiel, sous vide.

Le filtre de rétention 42 utilisé dans cette étape, incorporé dans la cartouche de filtration 40, a été choisi afin de séparer sélectivement les espèces contenues dans le filtrat issu de l'étape 10 et d'enrichir en microorganismes, tout en laissant passer autant de débris et espèces indésirables que possible.

Les critères guidant le choix du filtre, à savoir un pourcentage de perte des microorganismes dans le filtrat minimal et une absence de phénomènes susceptibles de limiter l'efficacité de la filtration, ont conduit les inventeurs à sélectionner un filtre en polycarbonate ou en nitrate de cellulose pour l'uniformité de structure de ses pores ainsi que son haut pouvoir d'adsorption non spécifique. Le filtre 42 ainsi choisi présente un diamètre moyen des pores situé entre 0,22 et 0,55 µm, avec une valeur préférée de l'ordre de 0,45 µm.

L'étape 14 du procédé d'extraction selon l'invention est relative à la lyse chimique et/ou mécanique des microorganismes contenus dans le rétentat issu de l'étape 12 dudit procédé. Comme indiqué précédemment, cette étape est réalisée *in situ,* c'est-à-dire au niveau même de la cartouche de filtration 40.

L'objectif recherché par les inventeurs était de mettre au point une procédure de lyse à la fois : (i) efficace ; (ii) adaptée à la biodiversité des mélanges complexes de départ, comprenant éventuellement des bactéries à Gram positif et des spores dont la lyse est, si l'on s'en tient aux méthodes classiques, longue et difficile ; et enfin (iii) adaptable à l'automatisation du procédé global.

Par lyse chimique, l'on entend l'action sur les cellules procaryotes d'un détergent, ledit détergent étant contenu par exemple dans un tampon de lyse comprenant notamment :
- du Tris-HCl (pH entre 7 et 9 , de préférence égal à environ 8 ; et concentration entre environ 25 et 400 mM, de préférence égale à environ 100 mM) ;
- le cas échéant, l'ensemble des trois composants suivants :
   - de l'EDTA (pH entre environ 7 et 9 de préférence égal à environ 8 ; et concentration entre environ 10 et 100 mM, de préférence égale à environ 40 mM) ;
   - du NaCl (de 50 à 800 mM, de préférence environ 200 mM) ;
   - du SDS (entre 0,5 et 8 % environ, de préférence environ 2 %),
   le mélange des quatre composants ci-dessus formant un tampon appelé TENS (Kuske *et al.* 1998. Appl. Environ. Microbiol. **64 :** 2463-2472) ;
   et
- du Chelex-100 (entre 3 et 60 % environ, de préférence environ 15 %).

Le traitement mécanique consiste en l'ultrasonication des cellules procaryotes fixées sur le filtre de rétention, ladite ultrasonication étant effectuée dans les mêmes conditions de tampon que la lyse chimique, dans un laveur à ultrasons et à des températures comprises entre 60 et 100°C, et de préférence avoisinant 70°C. Alternativement, ce traitement mécanique peut être réalisé à température ambiante et à sec, c'est-à-dire par fixation directe du récipient contenant la solution à lyser sur le générateur d'ultrason sans l'intermédiaire d'un bain, chaque cartouche de lyse étant directement posée sur une sonotrode indépendante.

Le traitement chimique, effectué alternativement ou subséquemment à la lyse mécanique, est administré à chaud. L'expression « à chaud » couvrant, au sens de la présente invention, des températures comprises entre 80 et 120°C, et de préférence avoisinant 100°C.

Selon un mode de réalisation préféré de la présente invention, l'étape 14 du procédé d'extraction comprend un traitement mécanique à température ambiante et à sec, suivi d'un traitement chimique, ledit traitements étant tels que décrits *supra.*

Des agents chaotropiques hautement dénaturants peuvent éventuellement être ajoutés au tampon de lyse, afin d'inhiber les activités DNAses et RNAses susceptibles de réduire le rendement d'extraction des acides nucléiques. Les agents chaotropiques propres à une utilisation dans le cadre du procédé selon l'invention comprennent, sans s'y limiter, le thiocyanate de guanidium, le chlorure de guanidium, l'urée, l'acide perchlorique, l'acide trichloroacétique, le thiocyanate de sodium, l'iodure de sodium, l'isothiocyanate de guanidium et le bromure d'hexadécyl triméthyl ammonium (CTAB).

Selon un mode de mise en oeuvre du procédé d'extraction objet de la présente invention (Figure 1, voie A), au cours de l'étape 16, la solution contenant les acides nucléiques d'intérêt est récupérée par aspiration à partir de la cartouche 40. Avantageusement, au moins un lavage de ladite cartouche 40 est effectué avec du tampon TE 1X, ceci afin de minimiser les pertes en acides nucléiques.

Ensuite, l'étape 18 vise à concentrer et purifier les acides nucléiques sur une colonne de silice. Il est ici fait appel à la chromatographie d'adsorption liquide-solide, mode de séparation basé sur la répartition des solutés entre l'adsorbant fixe et la phase liquide mobile. En l'occurrence, la silice est un adsorbant dipolaire. La fixation par adsorption est due à l'établissement de liaisons secondaires de surface, ou liaisons dipôle-ion, entre l'adsorbant et la molécule adsorbée.

L'étape 18 se décompose alors en au moins quatre sous-étapes :
181) la dilution de l'extrait d'acides nucléiques, notamment en présence d'éthanol absolu, ceci afin d'optimiser la liaison desdits acides nucléiques à la silice ;
182) le dépôt du mélange sur une colonne de silice ;
183) un ou plusieurs lavages de ladite colonne, suivis de son séchage ; et
184) l'élution des acides nucléiques retenus sur la colonne de silice.

Lors de la sous-étape 182, les acides nucléiques sont adsorbés sur la silice en présence de fortes concentrations de sels chaotropiques, lesquels éliminent l'eau des molécules hydratées du soluté.

Les polysaccharides, ainsi que les protéines ne s'adsorbent pas et sont éliminés au cours de la sous-étape 183.

Dans le cadre de la sous-étape 184, l'élution des acides nucléiques purifiés est effectuée en conditions de faible salinité.

De manière préférée, les sous-étapes 181 à 184 sont effectuées sous vide afin d'éviter tout recours à d'éventuelles centrifugations, notamment pour éluer le tampon de lavage ainsi que la solution d'acides nucléiques.

Selon un mode de réalisation préféré, le procédé d'extraction objet de la présente invention comprend des étapes préliminaires additionnelles, ayant pour but de faciliter les manipulations subséquentes du mélange complexe représenté, par exemple, par un échantillon alimentaire tel qu'un produit carné de type viande hachée, ou un produit laitier de type fromage.

Lesdites étapes préliminaires consistent à :
26) broyer l'échantillon complexe, notamment alimentaire ; et
28) prétraiter enzymatiquement et chimiquement le broyat ainsi obtenu.

L'invention concerne également une cartouche de filtration 40 utile pour la mise en oeuvre des étapes 12 et 14 (Figure 1, voie A) du procédé d'extraction des acides nucléiques de microorganismes.

Lorsque l'étape de concentration et purification est effectuée par chromatographie (étape 18), la cartouche 40 comprend un filtre de rétention 42 en polycarbonate, dont le diamètre moyen des pores est de préférence de 0,45 µm, inséré entre deux supports de filtration (grilles), le tout étant maintenu par deux bagues en polymère, tel que représenté sur la Figure 3. Cette cartouche peut être fermée de façon étanche afin d'être soumise aux traitements de lyse (Figure 4) ou, alternativement, le filtre de rétention est enchâssé dans une bague en polymère injecté. Dans ce cas, la pièce sur laquelle est posée la cartouche de filtration est dévissée du poste de filtration, et la cartouche est fermée de façon étanche sur sa face inférieure - c'est-à-dire la face qui n'est pas en contact avec les bactéries - par un couvercle. La face supérieure de la cartouche de filtration est fermée de façon étanche par un dispositif comprenant un entonnoir, un adaptateur, un filtre de clarification du lysat et un tube de 15 ml à fond conique. L'adaptateur est vissé sur l'entonnoir. Il contient le filtre de clarification du lysat, préalablement emboîté dans l'adaptateur. A l'extrémité libre de l'adaptateur, un tube collecteur de 15 ml à fond conique est vissé, tel que représenté sur la Figure 4.

L'invention a en outre pour objet un automate d'extraction des acides nucléiques de microorganismes contenus dans un mélange complexe, notamment alimentaire, ou dans de l'eau, ledit automate étant utile pour la mise en oeuvre du procédé d'extraction selon l'invention.

Par « automate », l'on désigne un dispositif ou appareil capable de mettre en oeuvre un procédé, d'en enchaîner les étapes essentielles de manière autonome, sans requérir l'assistance, l'intervention ou la surveillance d'un opérateur, si ce n'est éventuellement au niveau d'étapes ponctuelles et non essentielles, par exemple lorsqu'il est question d'assurer la connexion entre deux étapes essentielles.

L'invention vise également un dispositif pour l'extraction et la détection et, le cas échéant, la quantification des acides nucléiques de microorganismes contenus dans un mélange complexe, notamment alimentaire, ledit dispositif comprenant un automate d'extraction d'acides nucléiques conforme à la présente invention, couplé à un automate d'amplification en chaîne thermo-dépendante desdits acides nucléiques.

Dans un mode de réalisation préféré, l'automate d'amplification en chaîne thermo-dépendante incorporé dans le dispositif selon l'invention est de type rotatif.

Un automate d'amplification en chaîne thermo-dépendante de séquences d'acides nucléiques utilisable dans le cadre du dispositif selon la présente invention a été décrit dans le brevet US 6,821,771 délivré le 23 novembre 2004 bénéficiant de la priorité du brevet français FR 2812306 délivré le 14 janvier 2005. Un exemple d'un tel dispositif comprend notamment : (i) une cartouche comportant une pluralité de chambres réactionnelles, contenant des couples d'amorces pour l'amplification de séquences nucléotidiques spécifiques, auxquelles se trouve relié un réservoir d'alimentation en extrait d'acides nucléiques purifiés servant de matrices lors de ladite amplification ; (ii) une platine de chauffage dont au moins deux zones distinctes peuvent être portées à au moins deux températures différentes et maintenues constantes, chaque température correspondant ainsi à une étape donnée d'un cycle d'amplification, à savoir l'étape de dénaturation, d'hybridation ou d'élongation ; (iii) des moyens de déplacement relatif entre la cartouche et la platine, ledit déplacement assurant une exposition cyclique des chambres réactionnelles à la température de chacune des zones de la platine, ce qui permet d'enchaîner automatiquement les cycles de la réaction d'amplification se déroulant à l'intérieur desdites chambres.

La présente invention est illustrée, sans pour autant être limitée, par les figures suivantes :
Figure 1 : schéma du procédé d'extraction des acides nucléiques.
Figure 2 : schéma de l'automate d'extraction d'acides nucléiques. Y sont représentés les trois postes, à savoir le poste 80 effectuant les étapes 10 (optionnelle), 12, 16 et 18 du procédé d'extraction, les postes 82 et 84 effectuant l'étape 14.
Figure 3 : schéma d'une cartouche de filtration.
Figure 4 : schéma du montage pour effectuer l'étape 14 du procédé d'extraction
Figure 5 : schéma du montage pour effectuer l'étape 16 du procédé d'extraction.

### EXEMPLES

Le procédé d'extraction d'acides nucléiques objet de l'invention a été testé sur deux mélanges alimentaires complexes, à savoir un produit carné de type steak haché et un fromage à pâte molle commercialisé sous la marque Babybel^{®}, ainsi que sur un échantillon d'eau de consommation. Les deux mélanges alimentaires complexes illustrent la voie A de la figure 1 de la présente invention tandis que l'échantillon d'eau de consommation illustre la voie B de la figure 1 de la présente invention.

Les deux mélanges complexes ont été inoculés par des quantités connues de deux microorganismes différents (*Listeria monocytogenes* et *Salmonella ser. Enteritidis*), lesdits microorganismes étant choisis afin de vérifier l'efficacité d'extraction par le procédé selon l'invention des acides nucléiques de bactéries à Gram positif et à Gram négatif. L'échantillon d'eau de consommation a été inoculé par des quantités connues d'une souche de *Legionnella pneumophila* sérogroupe 1.

### I - Broyage de l'échantillon alimentaire

Conformément aux normes éditées et diffusées par l'Association Française de Normalisation (AFNOR) et la Fédération Internationale de Laiterie (FIL) actuellement en vigueur, l'échantillon alimentaire a été broyé à l'aide d'un stomacher afin de préparer une suspension-mère.

La prise d'essai de l'échantillon alimentaire, de 25 g, a été suspendue au 1/10^{ème} dans un diluant, conformément aux normes NF V 08-010 (mars 1996, Editions AFNOR, Paris, France), NF V 04-501 (avril 1998, Editions AFNOR) et FIL 122C : 1996 (décembre 1996, Editions FIL, Bruxelles, Belgique). Selon lesdites normes, la composition du diluant est fonction du type d'aliment considéré. Dans la présente espèce, le steak haché et le fromage ont respectivement été suspendus dans de l'eau peptonnée tamponnée et du citrate de sodium à 20 g/l.

Les suspensions ainsi obtenues ont été broyées pendant 3 minutes.

Des aliquotes de 10 ml de broyat, correspondant à 1 g d'aliment de départ, ont été prélevées aux fins de l'extraction des acides nucléiques.

Les matrices alimentaires peuvent également être traitées après un enrichissement en micro-organismes cibles dans des milieux de cultures définis, en fonction du micro-organisme recherché, par les normes AFNOR en vigueur. Dans ce cas, le prétraitement enzymatique est directement appliqué sur 10 ml de milieu enrichi.

### II - Prétraitement enzymatique et chimique de l'échantillon alimentaire

Cette étape permet d'hydrolyser les globules de matière grasse et les micelles protéiques, afin de s'affranchir des phénomènes tels le colmatage susceptibles d'abaisser le rendement des étapes ultérieures de filtration.

Les compositions des solutions de prétraitement ont été déterminées de manière empirique, à la lumière d'une étude bibliographique (Driehuis et Teernstra 1992. Neth. Milk Dairy J. **46 :** 209-215 ; Starbuck *et al.* 1992. Lett. Appl. Microbiol. 15 : 248-252 ; Yang et Lundahl 1994. Anal. Biochem. **218 :** 210-221 ; Rijpens *et al.* 1996. Appl. Environ. Microbiol. **62 :** 1683-1688).

Les adjuvants ajoutés à chaque aliquote de 10 ml étaient composés de 0,2 g de trypsine pour le broyat de steak haché, et 0,2 g de pancréatine, 10 mM d'EDTA, 5 % de Triton et 4 % d'acide cholique pour le broyat de fromage.

Les échantillons ont été incubés à 25 °C pendant 1 heure pour le broyat de steak haché, et à 30°C pendant 1 heure 30 min., pour le broyat de fromage. Cette étape d'incubation ne permet ni une croissance bactérienne significative, ni une lyse « prématurée » des cellules procaryotes, deux phénomènes susceptibles de nuire au rendement de la procédure.

### III - Microfiltration du broyat alimentaire (étape 10)

### III-1- Mise au point du protocole

Eu égard aux impératifs relatifs à l'évitement du colmatage, il a été choisi des filtres en nitrate de cellulose dont le diamètre moyen des pores est de 8 µm.

La microfiltration a été réalisée en mode plan, sous vide.

Chaque 10 ml de broyat alimentaire prétraité a été filtré. Selon qu'il s'agissait de steak haché ou de fromage, le dispositif de microfiltration a été lavé avec 40 ml de tampon TE 1X (Tris-HCl 10 mM pH 8,0 et EDTA 1 mM pH 8,0) ou 10 ml de Triton 0,5 % (dilué dans de l'eau distillée stérile) préchauffé à 50 °C, respectivement. Cette étape de lavage vise à limiter la perte de matériel, en particulier de bactéries d'intérêt, et diluer les échantillons de manière à éviter les colmatages, notamment lors de l'étape suivante de rétention sur filtre.

A l'issue de l'étape de microfiltration, 20 et 50 ml de filtrat contenant les bactéries cibles ont été récupérés pour chaque 10 ml de broyat initial de fromage et de steak haché, respectivement.

### III-2- Conditions de mise en oeuvre par l'automate

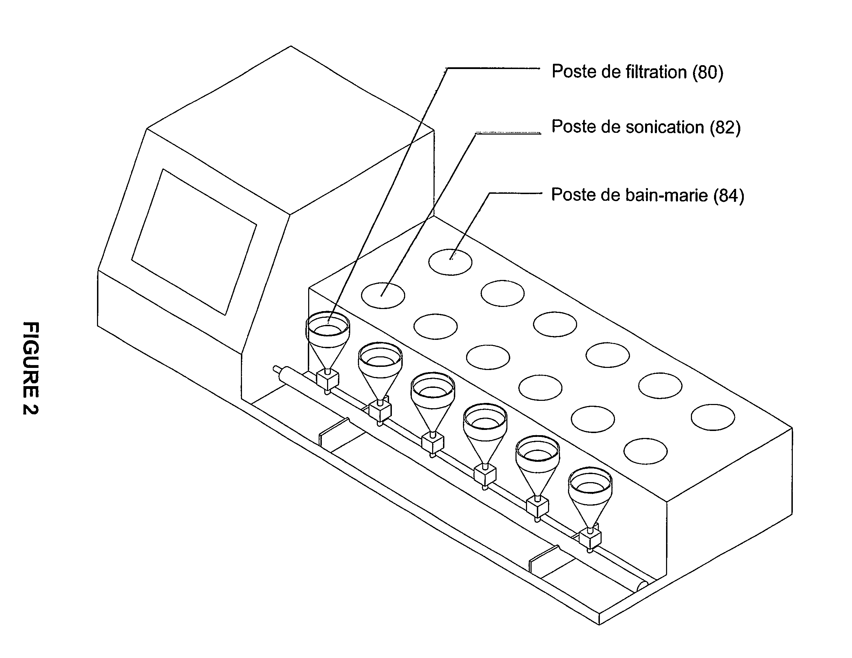

Cette étape est réalisée au niveau du poste filtration relié à la pompe, à l'aide d'un montage comportant, de haut en bas, un entonnoir à usage unique, une cartouche de préfiltration à usage unique, un cylindre en plastique et une cartouche de rétention à usage unique. Ce montage est connecté au poste de filtration 80 par l'intermédiaire d'une pièce en plastique (Figure 2).

La microfiltration est effectuée en mode plan.

Les liquides (broyats alimentaires additionnés de tampon de lavage) sont distribués manuellement.

### IV - Rétention des microorganismes (étape 12)

### IV-1- Mise au point du protocole

Après nombre de tests de filtres de rétention eu égard aux impératifs relatifs à l'évitement du colmatage, les meilleurs résultats ont été observés avec des filtres en polycarbonate dont le diamètre moyen des pores est de 0,45 µm.

Le filtrat de 40 ml a été filtré sur le filtre de rétention 42 ainsi sélectionné.

Le filtrat a été écarté et le filtre de rétention 42 placé dans un tube en polypropylène.

En ce qui concerne les échantillons d'eau de consommation, les étapes de broyage de l'échantillon et de prétraitement enzymatique et chimique de l'échantillon ne sont pas nécessaires (Figure 1, voie B). Conformément aux normes éditées et diffusées par l'Association Française de Normalisation (AFNOR) actuellement en vigueur, la prise d'essai de l'échantillon d'eau est comprise entre 250 ml et 2L, et préférentiellement 1 L. Cette prise d'essai est directement filtrée sur des filtres en polycarbonate dont le diamètre moyen des pores est de 0,45 µm afin de retenir les bactéries du genre *Legionnella.*

### IV-2- Conditions de mise en oeuvre par l'automate

La préfiltration et la rétention étant réalisées en enfilade, le filtrat de 40 ml est amené directement à l'issue de la préfiltration, par l'intermédiaire d'un cylindre, dans la cartouche de rétention 40 située à l'entrée du module 80 de l'extracteur (Figure 2).

Un bac de déchet est prévu, permettant d'évacuer le filtrat.

### V - Lyse des microorganismes cibles (étape 14)

### V-1- Mise au point du protocole

A l'issue de l'étape précédente, le filtre de rétention 42 a été déposé dans un tube en polypropylène, ce matériau étant proche du polymère requis pour fabriquer la cartouche de filtration 40.

Le rétentat fixé au filtre de rétention 42 a été suspendu dans 1 ml de tampon de lyse Tris-HCl 100 mM pH8,0 - Chelex-100 25%.

Le tube en polypropylène contenant le filtre de rétention 42 et le tampon de lyse a été placé dans un bain à sonication de 120 W. La sonication a été effectuée à 70°C pendant 40 min. et à 80% de la puissance maximale du sonicateur, soit une puissance comprise entre 80 et 120 W, et de préférence 100 W.

Le tube a ensuite été incubé au bain-marie pendant 10 min. à 100°C.

### V-2- Conditions de mise en oeuvre par l'automate

La pièce en plastique positionnée au niveau du poste filtration de la pompe à laquelle est reliée la cartouche de filtration, est dévissée. La cartouche de filtration est fermée sur sa face inférieure par un couvercle. 2 ml de tampon de lyse Tris-HCl 100 mM pH8,0 - Chelex-100 25% sont déposés sur la cartouche de filtration. Cette dernière est fermée sur sa face supérieure par un entonnoir sur lequel est vissé un adaptateur comprenant une colonne de clarification du lysat et un tube de 15 ml (figure 4).

Le montage pour effectuer la lyse est alors placé par l'expérimentateur sur une sonotrode au niveau du module 82, pendant 40 min., à une fréquence de 35 KHz. La cartouche est ensuite incubée par l'expérimentateur dans un bain-marie à sec au niveau du module 84, pendant 10 min. à 100°C.

### VI - Récupération des acides nucléiques (voies A et B, étape 16)

### VI-1- Mise au point du protocole

Le lysat bactérien contenu dans le tube en polypropylène est prélevé à l'aide d'une pipette de 1 ml et déposé dans un nouveau tube muni d'une colonne « Filter ». Le tube de lyse est ensuite rincé avec 1 ml de TE 1X, lequel est également prélevé et déposé sur la colonne « filter ». Le lysat est alors filtré sur la colonne « filter » par simple gravité. Le lysat filtré est prélevé et directement analysé par PCR ou concentré et purifié selon l'étape 18 de la voie B (Figure 1).

### VI-2- Conditions de mise en oeuvre par l'automate

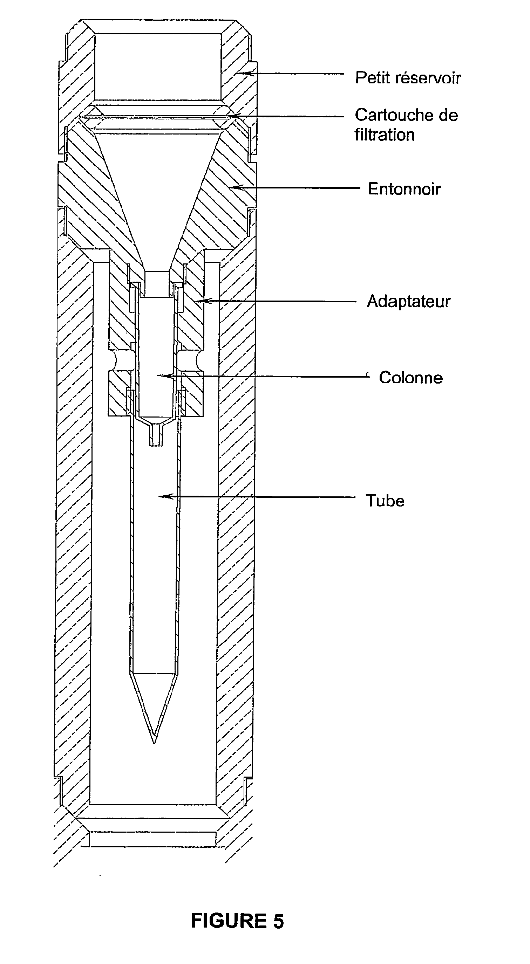

Le montage requis pour effectuer la lyse, contenant la cartouche de filtration 40, est placé par l'expérimentateur sur le poste filtration au niveau du module 80. Après avoir remplacé le couvercle par un petit réservoir (Figure 5), la pompe est connectée afin de filtrer le lysat à travers la colonne « filter ». L'expérimentateur dépose ensuite 1 ml de TE 1X sur la cartouche de filtration 40, le TE permet de rincer le filtre de rétention 42. Le tube en polypropylène contenant le lysat filtré est prélevé et l'ADN est directement analysé par PCR ou concentré et purifié selon l'étape 18 de la voie B (Figure 1).

### VII - Concentration et purification des acides nucléiques par chromatographie liquide-solide (voie B ; étape 18)

### VII - 1 - Mise au point du protocole

Les acides nucléiques ont été purifiés et concentrés à l'aide d'une colonne de silice, telle que disponible dans le système « Nucleospin Plant L » (Macherey-Nagel, Düren, Allemagne).

Une colonne de silice a été placée sur une chambre à vide permettant de traiter plusieurs échantillons simultanément. Ladite chambre était constituée d'une cuve en verre munie d'un manomètre et d'un couvercle en polypropylène, ledit couvercle pouvant recevoir, sur sa face externe, les colonnes et, sur sa face interne, les aiguilles à usage unique assurant le transfert de liquide.

Le cas échéant, le vide de chaque colonne pouvait être réglé à l'aide d'un robinet amovible.

Dans la cuve en verre, se trouvait un support permettant de placer des tubes en-dessous de chaque aiguille afin de récupérer les divers liquides (tampon de liaison et éthanol, solutions de lavage, éluat).

Les acides nucléiques (1 volume) ont été mélangés avec 1 volume de tampon de liaison (tampon « C4 » du système commercialisé sus-cité) et 1 volume d'éthanol absolu. Ce mélange a été agité vigoureusement pendant 30 secondes puis déposé sur une colonne telle que décrite ci-dessus.

La colonne de silice a d'abord été lavée avec 1 ml de tampon de lavage « CQW », puis avec 2 ml de tampon « C5 », lesdits tampons étant fournis dans le système « Nucleospin Plant L ».

Le tube placé dans la cuve en verre et contenant les déchets issus des étapes de lavage a été vidé.

Le vide a ensuite été appliqué pendant 12 minutes aux fins du séchage de la colonne.

200 µl de tampon « CE » préchauffés à 70°C ont incubé sur la colonne pendant 5 minutes.

Après élution sous vide dans un tube neuf, l'étape précédente a été répétée une fois. Les acides nucléiques ont alors été élués sous vide pendant 5 minutes.

Le volume d'acides nucléiques ainsi purifiés se situait entre 15 et 250 µl, et de préférence 200 µl.

### VII - 2 - Conditions de mise en oeuvre par l'automate

Au niveau du poste de filtration, l'adaptateur contenant un tube en polypropylène, dédié à la collecte du lysat filtré, et la colonne « filter » est dévissé. La colonne « filter » est éliminée et remplacée par une colonne de silice, nécessaire à la concentration et à la purification de l'ADN. A ce niveau, la colonne n'est pas conditionnée dans un tube de façon à permettre l'élimination du lysat (ne contenant plus l'ADN qui aura été retenu sur la silice) et des liquides de lavage, lesquels sont directement collectés dans un contenant à déchets.

Le lysat filtré contenu dans le tube en polypropylène (1 volume), est mélangé avec 1 volume de tampon de liaison (tampon « C4 » su système commercialisé par Macherey-Nagel) et 1 volume d'éthanol absolu. Ce mélange est agité vigoureusement pendant 30 secondes puis déposé au niveau du poste de filtration, sur la colonne de silice nouvellement installée. Après connexion de la pompe afin d'assurer la filtration du lysat sur la colonne de silice, l'ADN retenu sur la silice est lavé avec 1 ml de tampon de lavage « CQW » puis avec 2 ml de tampon « C5 », lesdits tampons étant fournis dans le système « Nucléospin Plant L » et distribués manuellement par l'expérimentateur.

Le vide est ensuite appliqué pendant 15 minutes aux fins du séchage de la colonne.

Un tube neuf dédié à la récupération de l'ADN concentré et purifié est vissé à l'extrémité libre de l'adaptateur.

200 µl de tampon TE 1X préchauffés à 70°C sont incubés sur la colonne pendant 5 minutes. Après élution sous vide pendant 1 minute dans le tube neuf, l'étape précédente a été renouvelée une fois.

Le volume d'acides nucléiques ainsi purifiés se situe entre 100 et 300 µl, et de préférence 200 µl.

## Revendications

1. Procédé d'extraction des acides nucléiques de microorganismes contenus dans un mélange complexe, notamment alimentaire, ou dans de l'eau, comprenant au moins les étapes suivantes :
(12) la rétention des microorganismes contenus dans le mélange sur un dispositif de type cartouche de filtration (40) ;
(14) la lyse des microorganismes *in situ* ; et
(16) la récupération des acides nucléiques à partir de la cartouche de filtration (40) sur un adaptateur contenant un filtre de clarification, lesdits acides nucléiques étant récupérés sous la forme d'un lysat filtré.

2. Procédé selon la revendication 1, dans lequel l'étape (12) est précédée d'une étape (10) de clarification du mélange complexe par filtration, ladite étape (12) étant alors effectuée sur le filtrat issu de l'étape (10).

3. Procédé selon la revendication 1 ou 2, dans lequel l'étape (10) de clarification du mélange complexe est réalisée à l'aide d'un filtre à usage unique en polycarbonate ou en nitrate de cellulose, et dont le diamètre moyen des pores est compris entre 1,2 et 12 µm, et de préférence égal à 5µm.

4. Procédé selon l'une quelconque des revendications 1 à 3, dans lequel l'étape (16) est suivie d'une étape (18) de concentration et purification des acides nucléiques par chromatographie d'adsorption liquide-solide.

5. Procédé selon l'une quelconque des revendications 1 à 4, dans lequel la cartouche (40) utilisée dans les étapes (12) et (14) comprend un filtre de rétention (42).

6. Procédé selon la revendication 4, dans lequel l'étape (18) est réalisée sur une colonne de silice.

7. Procédé selon la revendication 6, dans lequel l'étape (18) comprend au moins les sous-étapes suivantes :
(181) la dilution de l'extrait d'acides nucléiques ;
(182) le dépôt du mélange sur une colonne de silice ;
(183) au moins un lavage suivi du séchage de la colonne ; et
(184) l'élution des acides nucléiques retenus dans la colonne de silice.

8. Procédé selon la revendication 7, dans lequel les sous-étapes (181) à (184) sont réalisées sous vide.

9. Procédé selon la revendication 1, dans lequel l'étape (12) consiste en une microfiltration tangentielle, réalisée à l'aide du filtre de rétention (42) contenu dans la cartouche (40), ledit filtre (42) étant en nitrate de cellulose ou en polycarbonate et présentant un diamètre moyen des pores de 0,22 à 0,55 µm, et de préférence 0,45 µm.

10. Procédé selon la revendication 1, dans lequel l'étape (14) comprend un traitement de lyse chimique consistant en l'action à chaud, en particulier à 70 °C, du détergent contenu dans un tampon de lyse comprenant :
- du Tris-HCl (pH entre environ 7 et 9, de préférence égal à environ 8 ; et concentration entre environ 25 et 400 mM, de préférence égale à environ 100 mM) ;
- du Chelex-100 (entre 3 et 60 % environ, de préférence environ 15%) ;
- et, le cas échéant, l'ensemble des trois composants suivants :
- de l'EDTA (pH entre environ 7 et 9, de préférence égal à environ 8 ; et concentration entre environ 10 et 100 mM, de préférence égale à environ 40 mM) ;
- du NaCl (de 50 à 800 mM environ, de préférence environ 200 mM) ;
- du SDS (entre 0,5 et 8 % environ, de préférence environ 2 %).

11. Procédé selon l'une quelconque des revendications 1 à 10, dans lequel l'étape (14) comprend un traitement de lyse mécanique consistant en l'ultrasonication des microorganismes dans du tampon comprenant du TENS et du Chelex-100, de préférence du TENS 2X et du Chelex-100 15 %.

12. Procédé selon la revendication 11, dans lequel l'ultrasonication est effectuée à chaud, à environ 70°C.

13. Procédé selon l'une quelconque des revendications 1 à 10, dans lequel l'étape (14) comprend un traitement de lyse mécanique consistant en la sonication à sec des microorganismes.

14. Procédé selon la revendication 1, dans lequel l'étape (14) comprend un traitement de lyse mécanique selon l'une quelconque des revendications 11 à 13, et un traitement de lyse chimique selon la revendication 10.

15. Procédé selon la revendication 1, comprenant en outre les étapes préliminaires suivantes :
(26) le broyage de l'échantillon complexe, notamment alimentaire ; et
(28) le prétraitement enzymatique et chimique du broyat obtenu.

16. Automate d'extraction des acides nucléiques de microorganismes contenus dans un mélange complexe, notamment alimentaire, ou dans de l'eau pour la mise en oeuvre du procédé selon l'une quelconque des revendications 1 à 15, **caractérisé en ce qu'**il comprend au moins trois modules (80, 82, 84), le premier desdits modules effectuant les étapes (12), (16) et (18), les deuxième et troisième modules (82) et (84) effectuant l'étape (14) dudit procédé d'extraction.

17. Automate selon la revendication 16, **caractérisé en ce que** le premier module (80) est également capable d'effectuer l'étape (10) du procédé d'extraction selon l'une quelconque des revendications 1 à 15.

18. Dispositif pour l'extraction, la détection et, le cas échéant, la quantification des acides nucléiques de microorganismes contenus dans un mélange complexe, notamment alimentaire, ou dans de l'eau, comprenant un automate d'extraction d'acides nucléiques selon l'une quelconque des revendications 16 à 17, couplé à un automate d'amplification en chaîne thermo-dépendante desdits acides nucléiques.

19. Dispositif selon la revendication 18, dans lequel l'automate d'amplification en chaîne thermo-dépendante est de type rotatif.

## Claims

1. Method for extracting nucleic acids from microorganisms contained in a complex mixture, in particular of foods, or in water, said method comprising at least the following steps:
(12) retaining the microorganisms contained in the mixture on a filtration cartridge-type device (40);
(14) lysing the microorganisms *in situ*; and
(16) recovering nucleic acids from the filtration cartridge (40) on adapter containing a clarification filter, said nucleic acids being recovered under the form of a filtered lysate.

2. Method according to claim 1, in which step (12) is preceded by a step (10) for clarifying the complex mixture by filtration, said step (12) then being carried out on the filtrate obtained from step (10).

3. Method according to claim 1 or 2, in which step (10) for clarifying the complex mixture is carried out using a disposable polycarbonate or cellulose nitrate filter with a mean pore diameter in the range 1.2 to 12 µm, preferably 5 µm.

4. Method according to any one of claims 1 to 3, in which step (16) is followed by a step (18) for concentrating and purifying nucleic acids by liquid-solid adsorption chromatography.

5. Method according to any one of claims 1 to 4, in which the cartridge (40) used in steps (12) and (14) comprises a retention filter (42).

6. Method according to claim 4, in which step (18) is carried out on a silica column.

7. Method according to claim 6, in which step (18) comprises at least the following sub-steps:
(181) diluting the nucleic acid extract;
(182) depositing the mixture on a silica column;
(183) washing said column at least once, followed by drying of the column; and
(184) eluting nucleic acids retained on the silica column.

8. Method according to claim 7, in which sub-steps (181) to (184) are carried out under vacuum.

9. Method according to claim 1, in which step (12) consists in a tangential microfiltration carried out using a retention filter (42) contained in the cartridge (40), said filter (42) being formed from cellulose nitrate or polycarbonate and having a mean pore diameter of 0.22 to 0.55 µm, preferably 0.45 µm.

10. Method according to claim 1, in which step (14) comprises a chemical lysis treatment consisting of the action of heat, in particular at 70°C, of a detergent contained in a lysis buffer comprising:
• Tris-HCl (pH between 7 and 9, preferably about 8; and concentration between about 25 and 400 mM, preferably about 100 mM);
• Chelex-100 (between about 3% and 60%, preferably about 15%);
• and when appropriate, the following three components in combination:
o EDTA (pH between about 7 and 9, preferably about 8; and concentration between about 10 and 100 mM, preferably about 40 mM);
o NaCl (about 50 to 800 mM, preferably about 200 mM);
o SDS (between about 0.5% and 8%, preferably about 2%);

11. Method according to any one of claims 1 to 10, in which step (14) comprises a mechanical lysis treatment consisting in the ultrasonication of the microorganisms in a buffer comprising TENS and Chelex-100, preferably TENS 2X and Chelex-100 15%.

12. Method according to claim 11, in which ultrasonication is carried out hot, at about 70°C.

13. Method according to any one of claims 1 to 10, in which step (14) comprises a mechanical lysis treatment consisting in dry sonication of the microorganisms.

14. Method according to claim 1, in which step (14) comprises a mechanical lysis treatment according to any one of claims 11 to 13, and a chemical lysis treatment according to claim 10.

15. Method according to claim 1, further comprising the following preliminary steps:
(26) grinding the complex sample, in particular of food; and
(28) pre-treating enzymatically and chemically the obtained ground mixture.

16. Automaton for extracting nucleic acids from microorganisms contained in a complex mixture, in particular of foods, or in water, to carry out the method according to anyone to claim 1 to 15, **characterized in that** it comprises at least three modules (80, 82, 84), the first of said modules carrying out steps (12), (16) and (18), the second and third modules (82) and (84) carrying out step (14) of said extraction method.

17. Automaton according to claim 16, **characterized in that** the first module (80) is also capable of carrying out step (10) of the extraction method according to any one of claims 1 to 15.

18. A device for extracting, detecting and, if appropriate quantifying, nucleic acids of microorganisms contained in a complex mixture, in particular in food, or in water, comprising an automaton for extracting nucleic acids according to any one of claims 16 to 17, coupled to an automaton for heat-dependent chain amplification of said nucleic acids.

19. A device according to claim 18, wherein the automaton for heat-dependent chain amplification is rotary in type.

## Patentansprüche

1. Verfahren zur Extraktion von Nukleinsäuren von Mikroorganismen, die in einer komplexen, insbesondere alimentären, Mischung oder in Wasser enthalten sind, welches wenigstens die folgenden Schritte aufweist:
(12) das Zurückhalten von in der Mischung enthaltenen Mikroorganismen auf einer Vorrichtung der Art Filterkartusche (40);
(14) die Lyse von Mikroorganismen *in situ*; und
(16) die Rückgewinnung von Nukleinsäuren von der Filterkartusche (40) auf einem Adapter mit einem Klärungsfilter, wobei die Nukleinsäuren in Form eines gefilterten Lysats rückgewonnen werden.

2. Verfahren gemäß Anspruch 1, bei welchem dem Schritt (12) ein Schritt (10) zur Klärung der komplexen Mischung durch Filtration vorausgeht, wobei der Schritt (12) dann auf dem aus Schritt (10) hervorgegangenen Substrat durchgeführt wird.

3. Verfahren gemäß Anspruch 1 oder 2, bei welchem der Schritt (10) zur Klärung der komplexen Mischung mittels eines Filters zur einmaligen Benutzung aus Polycarbonat oder aus Cellulosenitrat durchgeführt wird, dessen Poren einen mittleren Durchmesser zwischen 1,2 und 12 µm, vorzugsweise 5 µm, aufweisen.

4. Verfahren gemäß einem der Ansprüche 1 bis 3, bei welchem dem Schritt (16) ein Schritt (18) der Konzentration und Reinigung von Nukleinsäuren durch Flüssig-Fest-Adsorptionschromatographie folgt.

5. Verfahren gemäß einem der Ansprüche 1 bis 4, bei welchem die bei den Schritten (12) und (14) verwendete Kartusche (40) einen Rückhaltefilter (42) aufweist.

6. Verfahren gemäß Anspruch 4, bei welchem der Schritt (18) auf einer Kieselerde-Kolonne durchgeführt wird.

7. Verfahren gemäß Anspruch 6, bei welchem der Schritt (18) wenigstens die folgenden Unterschritte aufweist:
(181) die Verdünnung des Nukleinsäureextrakts;
(182) die Ablagerung der Mischung auf der Kieselerde-Kolonne;
(183) wenigstens eine Waschung gefolgt von Trocknen der Kolonne; und
(184) die Elution der in der Kieselerde-Kolonne zurückgehaltenen Nukleinsäuren.

8. Verfahren gemäß Anspruch 7, bei welchem die Unterschritte (181) bis (184) unter Vakuum durchgeführt werden.

9. Verfahren gemäß Anspruch 1, bei welchem der Schritt (12) aus einer tangentialen Mikrofiltration besteht, die mittels eines in der Kartusche (40) enthaltenen Rückhaltefilters (42) durchgeführt wird, wobei der Filter (42) aus Cellulosenitrat oder aus Polycarbonat ist und einen mittleren Porendurchmesser von 0,22 bis 0,55 µm und vorzugsweise 0,45 µm aufweist.

10. Verfahren gemäß Anspruch 1, bei welchem der Schritt (14) eine chemische Lysebehandlung aufweist, die in der Wärmewirkung, insbesondere bei 70°C, des Reinigungsmittels besteht, das in dem Lysepuffer enthalten ist, welcher aufweist:
- Tris-HCl (pH zwischen 7 und 9, vorzugsweise gleich ungefähr 8; und Konzentration zwischen ungefähr 25 und 400 mM, vorzugsweise gleich ungefähr 100 mM);
- Chelex-100 (ungefähr zwischen 3 und 60%, vorzugsweise ungefähr 15%);
- und gegebenenfalls die Gruppe der folgenden drei Bestandteile:
- EDTA (pH zwischen 7 und 9, vorzugsweise gleich ungefähr 8; und Konzentration zwischen ungefähr 10 und 100 mM, vorzugsweise gleich ungefähr 40 mM);
- NaCl (ungefähr 50 bis 800 mM, vorzugsweise ungefähr 200 mM);
- SDS (ungefähr zwischen 0,5 und 8%, vorzugsweise ungefähr 2%).

11. Verfahren gemäß einem der Ansprüche 1 bis 10, bei welchem der Schritt (14) eine mechanische Lysebehandlung aufweist, die aus der Ultrabeschallung von Mikroorganismen in dem Puffer, der TENS und Chelex-100, vorzugsweise TENS 2X und Chelex-100 15% aufweist, besteht.

12. Verfahren gemäß Anspruch 11, bei welchem die Ultrabeschallung bei Wärme, ungefähr bei 70°C, durchgeführt wird.

13. Verfahren gemäß einem der Ansprüche 1 bis 10, bei welchem der Schritt (14) eine mechanische Lysebehandlung aufweist, die aus der Trockenbeschallung von Mikroorganismen besteht.

14. Verfahren gemäß Anspruch 1, bei welchem der Schritt (14) eine mechanische Lysebehandlung gemäß einem der Ansprüche 11 bis 13 und eine chemische Lysebehandlung gemäß Anspruch 10 aufweist.

15. Verfahren gemäß Anspruch 1, welches des Weiteren die folgenden vorbereitenden Schritte aufweist:
(26) das Zerkleinern der komplexen, insbesondere alimentären, Probe; und
(28) die enzymatische und chemische Vorbehandlung des erhaltenen zerkleinerten Guts.

16. Extraktionsautomat für Nukleinsäuren von Mikroorganismen, die in einer komplexen, insbesondere alimentären, Mischung oder in Wasser enthalten sind, für die Durchführung des Verfahrens gemäß einem der Ansprüche 1 bis 15, **dadurch gekennzeichnet, dass** dieser wenigstens drei Module (80, 82, 84) aufweist, wobei das erste der Module die Schritte (12), (16) und (18) durchführt, das zweite und dritte Modul (82) und (84) den Schritt (14) des Extraktionsverfahrens durchführen.

17. Automat gemäß Anspruch 16, **dadurch gekennzeichnet, dass** das erste Modul (80) gleichermaßen in der Lage ist, den Schritt (10) des Extraktionsverfahrens gemäß einem der Ansprüche 1 bis 15 durchzuführen.

18. Vorrichtung für die Extraktion, Detektion und gegebenenfalls Quantifizierung von Nukleinsäuren von Mikroorganismen, die in einer komplexen, insbesondere alimentären, Mischung oder in Wasser enthalten sind, welche einen Extraktionsautomaten für Nukleinsäuren gemäß einem der Ansprüche 16 und 17 aufweist, in Verbindung mit einem Amplifikationsautomaten in thermoabhängiger Kette der Nukleinsäuren.

19. Vorrichtung gemäß Anspruch 18, bei welcher der Amplifikationsautomat in thermoabhängiger Kette der drehenden Art ist.
